# FASCICULE DE BREVET EUROPEEN

(11) **EP 0 970 713 B1**
(45) Date de publication et mention de la délivrance du brevet: **28.09.2005**
(21) Numéro de dépôt: 99401683.0
(22) Date de dépôt: 06.07.1999
(51) Int. Cl.: A61M 16/00, A61N 1/36

(54) **Dispositif médical implantable actif permettant le traitement par électrostimulation du syndrome de l'apnée du sommeil**
Aktive implantierbare medizinische Vorrichtung zur Behandlung von Schlafapnoe durch Elektrostimulation
Active implantable medical device for treating sleep apnea with electrostimulation

(30) Priorité: 06.07.1998 FR 9808639
(43) Date de publication de la demande: 12.01.2000
(73) Titulaire: ELA MEDICAL, 92541 Montrouge (FR)
(72) Inventeur: Bonnet, Jean-Luc, 92120 Montrouge (FR)
(74) Mandataire: Dupuis-Latour, Dominique

(56) Documents cités:
- EP-A- 0 750 920
- EP-A- 0 770 407
- WO-A-84/02080
- WO-A-92/03983
- US-A- 5 024 222
- US-A- 5 485 851
- US-A- 5 713 933

## Description

L'invention concerne le diagnostic et le traitement du syndrome d'apnée du sommeil et son traitement par électrostimulation.

Le syndrome d'apnée du sommeil (SAS), plus précisément le syndrome d'apnée obstructive (et non centrale) du sommeil (SAOS) est une affection ayant généralement pour origine une obstruction des voies respiratoires, et qui est susceptible d'entraîner un certain nombre de troubles tels que respiration pénible et insuffisante, troubles du rythme cardiaque, hypertension.

Divers traitements ont été proposés par chirurgie, par voie médicamenteuse, ou encore par maintien d'une pression positive dans les voies respiratoires au moyen d'un masque facial porté pendant le sommeil.

Le US-A-5 485 851 (Medtronic) propose également de traiter le SAS par stimulation électrique neuro-musculaire. Ce document décrit un générateur d'impulsions implanté, piloté par un capteur permettant de suivre le rythme respiratoire et ainsi détecter la survenue d'une apnée. Lorsqu'une apnée est détectée, le générateur délivre une salve d'impulsions électriques à une électrode implantée de stimulation des muscles contrôlant les voies aériennes du patient. Le dispositif analyse l'état métabolique et fonctionnel du patient pour n'appliquer la stimulation que pendant les phases d'activité où un SAS est réellement susceptible d'apparaître. Le document US-A-5,485,851 représente l'état de la technique le plus proche et divulgue un dispositif médical implantable actif selon le préambule de la revendication 1.

L'un des buts de l'invention est de proposer une thérapie du SAS par des moyens différents, non plus par stimulation neurologique des muscles contrôlant les voies aériennes du patient mais, de façon caractéristique de l'invention, par une stimulation particulière du myocarde en cas de SAS détectée.

L'invention s'applique ainsi particulièrement bien au traitement du SAS chez des patients qui sont déjà porteurs d'un stimulateur cardiaque (car il ne sera pas nécessaire d'implanter un dispositif spécifique pour le traitement du SAS). Mais on peut également prévoir d'implanter un stimulateur cardiaque selon l'invention dans le seul but de traiter le SAS, si nécessaire ; la technique d'implantation des stimulateurs est en effet une technique très bien maîtrisée et répandue, et la mise en place d'un dispositif selon l'invention ne nécessite, du point de vue du chirurgien, aucun changement dans le *modus operandi* auquel il est accoutumé.

Plus précisément, le dispositif de l'invention est un dispositif médical implantable actif comprenant : des moyens de stimulation cardiaque ; des moyens de mesure de l'activité respiratoire du patient; et des moyens de détermination d'un état d'activité, cet état étant susceptible de prendre, en fonction de critères prédéterminés, une valeur représentative d'un état de sommeil du patient, des moyens d'analyse, pour déterminer la survenue d'une apnée en fonction du signal délivré par les moyens de mesure, et des moyens de commande des moyens de stimulation, aptes à commander l'application sélective au patient d'un stimulus électrique cardiaque en cas de détection d'une apnée, ces moyens de commande n'étant activables que pendant les phases de sommeil et étant inhibés dans le cas contraire, de manière à permettre le traitement par électrostimulation du syndrome de l'apnée du sommeil chez le patient.

Selon diverses caractéristiques avantageuses :
- les moyens de stimulation cardiaque sont des moyens aptes, en cas de survenue d'une apnée, à accélérer la fréquence de battement du myocarde par rapport au rythme sinusal naturel du patient ;
- les moyens d'analyse détectent la survenue d'apnées successives au cours d'une phase de sommeil et déterminent la survenue d'un syndrome d'apnée du sommeil lorsque le nombre d'apnées détectées pendant une période de temps donnée dépasse un seuil prédéterminé ;
- les moyens de détermination d'un état d'activité analysent le signal délivré par lesdits moyens de mesure de l'activité respiratoire du patient et/ou par des moyens de mesure auxiliaires, distincts des moyens de mesure de l'activité respiratoire du patient;
- les moyens de mesure de l'activité respiratoire du patient comprennent un capteur de ventilation-minute et/ou un capteur de mesure de la saturation en oxygène du sang.

D'autres caractéristiques de l'invention apparaîtront à la lecture de la description détaillée ci-dessous d'un exemple de réalisation, en référence à la figure unique qui montre un signal représentatif du rythme respiratoire du patient, respectivement en l'absence de trouble et au moment de la survenue d'une apnée.

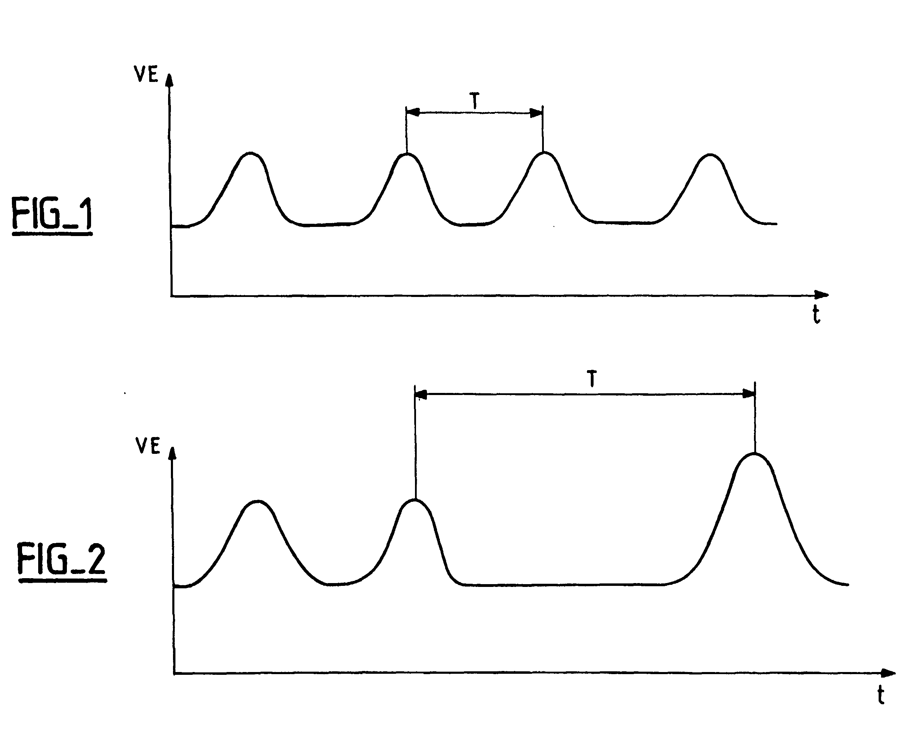

La figure 1 représente l'évolution du rythme respiratoire d'un patient pendant le sommeil, représenté ici par l'évolution au cours du temps du signal de ventilation-minute (signal VE, dit également signal MV), qui est un paramètre à prépondérance physiologique obtenu par une mesure intrathoracique d'impédance. Mais, bien que le signal de ventilation-minute soit généralement le plus aisé à mettre en oeuvre pour le suivi du rythme respiratoire du patient, d'autres signaux provenant d'autres types de capteurs peuvent être utilisés en variante ou en complément du capteur de ventilation-minute, par exemple un capteur de mesure de la saturation en oxygène dans le sang.

La mesure de la ventilation-minute est en elle-même bien connue ; elle est opérée entre deux électrodes disposées dans la cage thoracique, ou entre une électrode (par exemple une électrode de stimulation, si le dispositif implanté est un stimulateur cardiaque) et un boîtier du dispositif médical implanté. L'impédance est mesurée par injection d'un courant constant de quelques centaines de microampères, à une fréquence de quelques hertz, typiquement 8 Hz. Cette technique est par exemple décrite par Bonnet JL et coll., Measurement of Minute-Ventilation with Different DDDR Pacemaker Electrode Configurations, *PACE,* Vol. 21, 98, Part 1, et elle est mise en oeuvre dans les appareils *Chorus RM 7034* d'ELA Médical.

On peut déterminer à partir de ce signal une période respiratoire T (figure 1), définie comme étant le temps séparant deux pics d'impédance Les pics correspondent à de fortes impédances obtenues lors de l'inspiration (poumons remplis d'air), et la décroissance de l'impédance correspond à une phase expiratoire.

La figure 2 illustre le signal de ventilation-minute relevé chez des patients souffrant d'apnée du sommeil. Ces patients ont des phases expiratoires normales, car la pression pulmonaire est suffisante pour vaincre l'obstruction ; en revanche, l'inspiration est anormale car les poumons ne peuvent pas se remplir d'air. On peut alors observer, comme illustré sur cette figure 2, un allongement important de la période respiratoire T après une expiration.

La première étape consiste à diagnostiquer une apnée du sommeil.

Une apnée est définie classiquement comme une pause respiratoire de durée supérieure à dix secondes, phénomène qu'il est aisé de détecter par suivi du signal de ventilation-minute.

De plus, cette pause doit survenir pendant une phase de sommeil du patient, car une apnée en état d'éveil ne peut en aucun cas être à l'origine d'un SAS.

Pour respecter ce dernier critère, l'invention propose de prévoir une discrimination entre le sommeil et l'éveil du patient, afin de n'appliquer une thérapie que pendant les phases de sommeil et inhiber tout traitement si l'apnée survient pendant une phase d'éveil, car dans ce cas elle n'est normalement pas pathologique.

La période de sommeil est diagnostiquée bien entendu de façon automatique, soit à partir du signal délivré par le capteur de suivi du rythme respiratoire du patient, soit par un capteur distinct, par exemple un capteur d'activité mesurant un paramètre à prépondérance physique tel que l'accélération mesurée par un capteur interne au boîtier.

Le EP-A-0 719 568 (ELA Médical) décrit en particulier un procédé de détermination d'un "critère d'activité de capteur" permettant d'opérer une distinction entre des phases de repos (nocturne ou diurne) du porteur de l'appareil et d'autres phases d'activité, notamment à partir d'un capteur de ventilation-minute.

Les EP-A-0 750 920 (ELA Médical) et EP-A-0 770 407 (ELA Médical) décrivent, quant à eux, des dispositifs médicaux utilisant les informations combinées d'un capteur physiologique et d'un capteur physique, notamment d'un capteur de ventilation-minute et d'un accéléromètre, pour déterminer un état d'activité ou de repos du patient.

Ayant ainsi diagnostiqué une apnée et confirmé que celle-ci est bien une apnée du sommeil, on procède au calcul de l'indice d'apnée.

Pour ce faire, lorsque cet indice dépasse un seuil prédéterminé, par exemple plus de dix apnées par heure (ce nombre pouvant être éventuellement programmable en fonction du patient), on définit la présence d'un SAS.

Dès qu'un SAS est diagnostiqué, une stimulation électrique est alors appliquée au patient pour compenser les effets néfastes du SAS.

Cette stimulation est, selon l'invention, une stimulation cardiaque, par exemple par accélération de la fréquence de battement du myocarde, pour compenser les effets du SAS. Cette stimulation cardiaque sera appliquée dès qu'un SAS est diagnostiqué, en augmentant la fréquence de stimulation de quelques coups par minute (typiquement +10 cpm) par rapport au rythme sinusal naturel du patient.

## Revendications

1. Un dispositif médical implantable actif comprenant :
- des moyens de stimulation,
- des moyens de mesure de l'activité respiratoire du patient, et
- des moyens de détermination d'un état d'activité, cet état étant susceptible de prendre, en fonction de critères prédéterminés, une valeur représentative d'un état de sommeil du patient,
- des moyens d'analyse, pour déterminer la survenue d'une apnée en fonction du signal délivré par les moyens de mesure, et
- des moyens de commande des moyens de stimulation, ces moyens de commande n'étant activables que pendant les phases de sommeil et étant inhibés dans le cas contraire,
de manière à permettre le traitement par électrostimulation du syndrome de l'apnée du sommeil chez le patient,
dispositif **caractérisé en ce que** les moyens de stimulation sont des moyens de stimulation cardiaque et que les moyens de commande sont aptes à commander l'application sélective au patient d'un stimulus électrique cardiaque en cas de détection d'une apnée.

2. Le dispositif de la revendication 1, dans lequel les moyens de stimulation cardiaque sont des moyens aptes, en cas de survenue d'une apnée, à accélérer la fréquence de battement du myocarde par rapport au rythme sinusal naturel du patient.

3. Le dispositif de la revendication 1, dans lequel les moyens d'analyse détectent la survenue d'apnées successives au cours d'une phase de sommeil et déterminent la survenue d'un syndrome d'apnée du sommeil lorsque le nombre d'apnées détectées pendant une période de temps donnée dépasse un seuil prédéterminé.

4. Le dispositif de la revendication 1, dans lequel les moyens de détermination d'un état d'activité analysent le signal délivré par lesdits moyens de mesure de l'activité respiratoire du patient.

5. Le dispositif de la revendication 1, dans lequel les moyens de détermination d'un état d'activité analysent le signal délivré par des moyens de mesure auxiliaires, distincts desdits moyens de mesure de l'activité respiratoire du patient.

6. Le dispositif de la revendication 1, dans lequel les moyens de mesure de l'activité respiratoire du patient comprennent un capteur de ventilation-minute.

7. Le dispositif de la revendication 1, dans lequel les moyens de mesure de l'activité respiratoire du patient comprennent un capteur de mesure de la saturation en oxygène du sang.

## Patentansprüche

1. Aktive implantierbare medizinische Vorrichtung, aufweisend:
- Stimulationsmittel,
- Mittel zur Messung der Aternaktivität des Patienten, und
- Mittel zur Bestimmung eines Aktivitätszustands, wobei dieser Zustand abhängig von vorbestimmten Kriterien einen Wert einnehmen kann, der für den Schlafzustand des Patienten repräsentativ ist,
- Analysemittel, um das Auftreten einer Apnoe in Abhängigkeit des durch die Messmittel gelieferten Signals zu bestimmen, und
- Mittel zur Steuerung der Stimulationsmittel, wobei diese Steuermittel nur während der Schlafphasen aktivierbar sind und im gegenteiligen Fall gehemmt sind,
um die Behandlung durch Elektrostimulation des Schlafapnoesyndroms beim Patienten zu ermöglichen, wobei die Vorrichtung **dadurch gekennzeichnet ist, dass** die Stimulationsmittel Herzstimulationsmittel sind und die Steuermittel angepasst sind, die selektive Anwendung eines elektrischen Herzstimulus auf den Patienten im Fall des Entdeckens einer Apnoe zu steuern.

2. Vorrichtung gemäß Anspruch 1, in der die Mittel zur Herzstimulation Mittel sind, die dazu geeignet sind, im Fall des Auftretens einer Apnoe die Herzschlagfrequenz im Vergleich zum natürlichen Sinusrhythmus des Patienten zu beschleunigen.

3. Vorrichtung gemäß Anspruch 1, in der die Analysemittel das Auftreten aufeinander folgender Apnoen im Laufe einer Schlafphase entdecken und das Auftreten eines Schlafapnoesyndroms feststellen, wenn die Anzahl der entdeckten Apnoen während einer gegebenen Zeitdauer einen vorbestimmten Schwellenwert überschreitet.

4. Vorrichtung gemäß Anspruch 1, in der die Mittel zur Bestimmung eines Aktivitätszustands das von den Mitteln zur Messung der Aternaktivität des Patienten gelieferte Signal analysieren.

5. Vorrichtung gemäß Anspruch 1, in der die Mittel zur Bestimmung eines Aktivitätszustands das Signal analysieren, das von zusätzlichen Messmitteln geliefert wird, die von den Mitteln zur Messung der Aternaktivität des Patienten verschieden sind.

6. Vorrichtung gemäß Anspruch 1, in der die Mittel zur Messung der Aternaktivität des Patienten einen Atemminutenvolumensensor umfassen.

7. Vorrichtung gemäß Anspruch 1, in der die Mittel zur Messung der Aternaktivität des Patienten einen Sensor zur Messung der Sauerstoffsättigung des Blutes umfassen.

## Claims

1. An active implantable medical device including:
- stimulating means,
- means for measuring the respiratory activity of the patient, and
- means for determining a state of activity, this state being likely to take, according to predetermined criteria, a value representative of a state of sleep of the patient,
- analysing means, for determining the occurrence of an apnea in response to the signal issued by the measuring means, and
- means for controlling the stimulating means, said controlling means being enabled only during the sleep phases and being inhibited in the contrary case,
whereby permitting electrostimulation treatment of sleep apnea syndrome with the patient,
said device being **characterised in that** the stimulating means are cardiac stimulation means and the controlling means are means for controlling the selective application to the patient of a cardiac electrical stimulus in the event of a detection of an apnea.

2. The device of claim 1, wherein the cardiac stimulating means are means for, in the event of an the occurrence of an apnea, accelerate the cardiac stimulation rate of the heart with respect to the natural sinusal rate of the patient.

3. The device of claim 1, wherein the analysing means detect the occurrence of successive apnea during a sleep phase and determine the occurrence of a sleep apnea syndrome when the number of apnea detected during a given period of time exceeds a predetermined threshold.

4. The device of claim 1, wherein the means for determining a state of activity analyse the signal issued by said means for measuring the respiratory activity of the patient.

5. The device of claim 1, wherein the means for determining a state of activity analyse the signal issued by auxiliary measurement means, separate form said means for measuring the respiratory activity of the patient.

6. The device of claim 1, wherein the means for measuring the respiratory activity of the patient include a minute-ventilation sensor.

7. The device of claim 1, wherein the he means for measuring the respiratory activity of the patient include a sensor for measuring the oxygen saturation of the blood.
